# EUROPEAN PATENT SPECIFICATION

(11) **EP 3 099 249 B1**
(45) Date of publication and mention of the grant of the patent: **05.07.2023**
(21) Application number: 15701915.9
(22) Date of filing: 15.01.2015
(51) Int. Cl.: A61B 17/06, A61B 17/00

(54) **SELF-RETAINING SUTURE DEVICE WITH ASYMMETRIC RETAINERS**
SELBSTHALTENDE NAHTVORRICHTUNG MIT ASYMMETRISCHEN HALTERN
DISPOSITIF DE SUTURE AUTOSTATIQUE AVEC ÉLÉMENTS DE CONTENTION ASYMÉTRIQUES

(30) Priority: 27.01.2014 US 201461931727 P
(43) Date of publication of application: 07.12.2016
(73) Proprietor: Ethicon, Inc, Somerville, NJ 08876 (US)
(72) Inventor: LINDH, SR. David C., Flemington, New Jersey 08822 (US); ROUSSEAU, Robert A., Ottsville, Pennsylvania 18942 (US)
(74) Representative: Carpmaels & Ransford LLP
(86) International application number: PCT/US2015/011530
(87) International publication number: WO 2015/112416

(56) References cited:
- WO-A2-03/017850
- WO-A2-2008/041265
- WO-A2-2009/097556
- WO-A2-2011/139916
- US-A1- 2009 210 006

## Description

### Cross-Reference to Related Applications

This application claims priority to U.S. Provisional Application Serial No. 61/931,727, filed January 27, 2014.

### Field of the Invention

The present invention relates to self-retaining suture devices. In particular the invention relates to sutures having a plurality of asymmetric retainers along a portion of its axial length.

### Background of the Invention

Various surgical methods employing sutures have been used in the past for closing or binding together wounds in human or animal tissue, such as skin, muscles, tendons, internal organs, nerves, blood vessels, and the like. More specifically, the surgeon may use a surgical needle with an attached conventional suture (which can be a smooth monofilament or can be a multi-filament) to pierce the tissue alternately on opposing faces of the wound and thus sew the wound closed. Whether the wound is accidental or surgical, loop stitching is the method often used, especially for surface wounds. The surgical needle is then removed and the ends of the suture are tied, typically with at least three overhand throws to form a knot.

Self-retaining sutures, also sometimes referred to as barbed sutures, have offered numerous advantages over closing wounds with conventional sutures. A self-retaining suture includes an elongated body that has one or more spaced retainers, which project from the body surface along the body length. The retainers are arranged to allow passage of the suture in one direction through tissue but resist movement of the suture in the opposite direction. Accordingly, self-retaining sutures do not have to be knotted, the requirement for a surgical follower to maintain tension on a continuous stitch, as is done in the application of conventional sutures, is also eliminated.

A self-retaining suture should have suitable tensile strength, allowing it to be pulled through tissue by a surgeon during use. However, when pulled in the opposing direction, the retainers should be capable of sufficiently holding tissue and resisting movement of the suture in that opposing direction. Thus, the self-retaining sutures must have sufficient holding strength.

Self-retaining sutures should include retainers that allow for the smooth passage through tissue in a first direction but resist movement in the reverse direction due to the retainers ability to latch onto tissue. The cross-section of the suture and needle are important factors to consider in forming a suitable self-retaining suture. In addition, particularly in the age of robotic surgery, allowing for a suture device to provide a tactile or other sensation as it is pulled through tissue is particularly advantageous.

During the course of tissue passage towards the distal end of a conventional barbed suture (the "distal end" being the end furthest away from the insertion end of a suture), the retainers are collapsed inward due to the pressure of the surrounding tissue within the needle tract. However, the tract formed by the needle at the insertion of the suture may be larger in cross sectional area or diameter than the suture that is pulled through the tissue. The tract is larger due to the fact that the needle is larger in diameter than the fiber that is attached on most pre-needled surgical sutures. As a result of this mismatch in suture and needle diameter, coupled with the flexible collapse of the retainers, the retainer engagement during reverse tensioning may not occur as intended. Further, since the cut barb geometries in conventional barbed sutures are generally cut into the surface of the fiber from the tip to the base of the retainer, the tips of the barbs that are produced may be produced with a slightly bent tip, whereby the tip of the barb is actually oriented towards the central axis of the suture, away from the tissue to be engaged thereby exacerbating the potential for lack of tissue engagement. This results in a self-retaining suture that may not be retained in the tissue.

Conventional methods of forming retainers on a filament may result in retainers that have varying cross-sectional areas or too large a cross-sectional area due to retainers that cannot significantly collapse or deflect when pulled through tissue. While this may provide for suitable retention when the suture is pulled in an opposing direction, it may damage tissue when pulled in the insertion direction.

As self-retaining sutures have been utilized in applications where the working environment is more confined, additional functional requirements of the sutures have also been identified. The provision of surgical optical cues is necessary in use within the confines of an endoscopic environment when robotic systems are utilized to place the devices. Possible optical cues available to the robotic physician include the motion of tissue as the suture is pulled through the tissues due to small amounts of localized drag exerted by the individual retainers on the tissues during passage, tissue blanching, tissue tract quality and wound apposition. When the retainers are placed through the use of open or laparoscopic methods, it is important for the device to provide features that will indicate retainer engagement as can be felt through the handling instrumentation, commonly referred to as tactile feedback.

Previous methods of forming retainers on a suture have traditionally included the use of cutting blades that provide a smooth, even cut through the suture, starting the cut at the tip of the retainer and ending at the base of the retainer (where the retainer meets the suture filament). While these previous methods are useful in forming smooth and even retainers in a suture, the present application describes an improved method of forming retainers in a suture where the retainers have an asymmetric geometry. Whilst no claim is directed to this method *per se,* the method described herein is considered as useful for understanding the invention The method cuts retainers by starting at the base of the retainer and slicing through the filament ending at the tip of the retainer. The formation of a suture with asymmetric retainers can result in a device that has suitable tensile strength, provides tactile or other feedback during use, and gives increased holding in tissue.

WO 2009/097556 A2 describes an apparatus and method for cutting retainers on a continuous strand. The apparatus may include a rotary head member configured to be rotatably driven about a longitudinal axis. The rotary head member may include a center hole substantially coincident with the longitudinal axis and configured to receive the strand. A retainer forming member may be supported on the rotary head member and may include a cutting edge directed substantially inward toward the longitudinal axis. When the rotary head member rotates about the longitudinal axis and the strand is continuously pulled through the center hole along the longitudinal axis, the cutting edge may intermittently or continuously cut retainers about an outer surface of the strand.

WO 2011/139916 A2 describes a self-retaining suture with a suture thread less than 1mm nominal diameter. A plurality of retainers is cut into the suture thread using a high accuracy retainer cutting machine. The retainer cutting machine has sufficient accuracy and repeatability to cut consistent and effective retainers at high density on suture threads less than 1mm nominal diameter.

WO 03/017850 A2 describes a method of making a barbed suture by varying the blade geometry and/or the movement of the blade when cutting a suture. The method can also be accomplished with a cutting device to create a plurality of barbs on the exterior of a surgical suture. The barbs produced using the method with the cutting device can be the same or random configurations.

WO 2008/041265 A2 describes a thread provided with barbs for surgical purposes using threads of polyhedral or stochastic shape. The barbs are provided on the threads by making a cut in a point corresponding to an edge of the polyhedral or stochastic shape for each barb, preferably by means of techniques of laser etching that also enables barbs with a double tip to be obtained.
US 2009/210006 A1 describes a compound barb medical device which includes an elongated body having at least one barb formed along the length of the body, the barb defining an inner surface with a first portion disposed at a first orientation relative to a longitudinal axis of the elongated body, and a second portion disposed at a second orientation relative to the longitudinal axis. Optionally, the barb defines a third portion disposed at a third orientation relative to the longitudinal axis. Summary of the Invention

In one embodiment of the present invention, there is provided a self-retaining suture including a filament having a proximal end and a distal end, with an elongated body therebetween, the elongated body having a central axis and an outer perimeter; and a plurality of retainers formed in the elongated body of said filament, wherein each retainer has a distal tip, a proximal base where the undersurface of the retainer meets the elongated body, a first side portion connecting one end of said proximal base and said distal tip, and a second side portion connecting the other end of said proximal base and the distal tip, said first side portion having a shorter length than said second side portion; and wherein said proximal base of each retainer has an arcuate shape.

Certain methods are described with reference to the self-retaining suture of the present invention. Whilst no claim is directed to these methods *per se,* the method is considered as useful for understanding the invention. The method of forming a self-retaining suture includes the steps of positioning a filament into an apparatus, the filament including a body with a central axis and an outer perimeter, and the apparatus including a cutting apparatus, the cutting apparatus including a blade rotatably secured to a base and configured to contact the filament; activating the cutting apparatus, whereby activation moves the blade along an axis of rotation into contact with the body of the filament; cutting into the body of the filament such that the blade enters the interior of the body and traverses at an angle out of the body, forming a retainer in the body of the filament, where the retainer has a distal tip, a proximal base where the undersurface of the retainer meets the elongated body, a first side portion connecting one end of said proximal base and the distal tip, and a second side portion connecting the other end of said proximal base and the distal tip, said first side portion having a shorter length than said second side portion; and wherein said proximal base of each retainer has an arcuate shape.

### Brief Description of the Drawings

Figure 1 is a side view of one embodiment of a self-retaining suture of the present invention.
Figure 2 is a close-up view of the embodiment of Figure 1.
Figure 3 is a top view of a retainer of the embodiment of Figure 1.
Figure 3A is a cross-sectional view of Figure 3 taken along Section A-A.
Figure 4 is a side view of the retainer of Figure 3.
Figure 5 is a perspective view of one embodiment of a cutting method described herein.
Figure 6 is a side-view of the cutting method of Figure 5.
Figure 7 is a depiction of the cutting process described herein.
Figure 8 is a top view of one embodiment of a self-retaining suture of the present invention.
Figure 8A is a cross-sectional view of a cut made into a triangular suture.
Figure 9 is a depiction of a cutting apparatus and the path that the cutting apparatus may take in forming a self-retaining suture.
Figure 10 is a depiction of the cutting apparatus of Figure 9 showing its cutting pathway.
Figure 11 is a depiction of a manual cutting apparatus.
Figure 12 is a schematic depiction of the components of Figure 11.
Figure 13 is a side view of the cutting apparatus of Figure 11 in use.
Figure 14 is a top view of the cutting apparatus of Figure 11 in use.
Figure 15 is a close-up top view of a retainer formed by a manual cutting apparatus.
Figure 16 is a view of a suture formed by a manual cutting apparatus.
Figures 17-18 are plots comparing the force required to pull a conventional barbed suture (Figure 17) and a self-retaining suture of the present invention (Figure 18) through bladder tissue.
Figures 19-20 are plots comparing the effect of reverse tensioning on the force required to pull a conventional barbed suture (Figure 19) and a self-retaining suture of the present invention (Figure 20) through tissue in the reverse direction in swine bladder.

### Detailed Description

The present invention relates to sutures. The suture includes a filamentous strand of material, which may be made of a single material or may be a blended material. The material may be absorbable or degradable, or the material may be nonabsorbable. As is well known, conventional sutures can be of non-absorbable material such as silk, nylon, polyester, polypropylene, or cotton, or can be of bio-absorbable material such as glycolic acid polymers and copolymers or lactic acid polymers and copolymers. Of course, other materials, including metals, such as stainless steel as is utilized in sternal closure, may be used as desired. Although monofilament sutures are preferred, the suture may be multi-filament, such as a braided suture, or may be combinations of a monofilament suture with an outer region comprising braids or other materials. The suture filament may be produced via any conventional means including, for example, extrusion.

The suture filament includes a body having a length as measured along its central axis and an outer surface surrounding the central axis. The filament body may take any desired cross-sectional shape or configuration. For example, the filament may be cylindrical, or it may have any desired cross-sectional shape, such as triangular, rectangular, or other geometric shapes. As used herein, the cross-section of a suture is intended to refer to the shape provided by an imaginary plane crossing the suture body at a perpendicular angle to the central axis of the suture. The thickness of the suture is determined by measuring the diameter of the suture across its cross-section or, in the case of non-circular cross sections, by a line crossing the central axis of the cross-sectional plane.

The suture has a proximal end and a distal end with the body therebetween. The suture may be a unidirectional suture, where the proximal end is the leading end of the suture to be inserted into tissue and the distal end is the trailing end, which may optionally include a termination feature, such as an anchor, loop, knot or other securement end. The proximal end may include a needle or other insertion mechanism secured thereto for insertion into tissue. The suture may optionally be a bidirectional suture, having a needle or other insertion means at both the proximal end and distal end.

The suture body includes at least one, and more desirably a plurality of retainers (sometimes referred to as "barbs") extending outwardly from the surface of the suture body along at least a portion of its axial length. In some embodiments, the plurality of retainers are included from the proximal end to the distal end of the suture body, and in other embodiments, the retainers are only present along a particular section or sections of the suture body. The retainer tip is pointed axially away from the insertion end of the suture. That is, if the proximal end of a unidirectional suture includes a needle for insertion, the unattached or free end of the retainer will face the distal end of the suture. This allows the suture to be pulled through tissue in the direction of insertion without substantial resistance by the retainer, but when pulled in the opposing direction, the retainer's tip engages tissue and restricts movement in the opposing direction. In bidirectional sutures, there are at least two regions of retainers, each facing opposing directions, which may be separated by a retainer-free region of the suture or they may converge at a central axial point on the suture. Bidirectional sutures are known by those of skill in the art and are not discussed in detail herein, but the sutures and methods described herein may be used to form bidirectional sutures if desired.

The retainers may be formed through any desired means, and as will be described in greater detail below, in preferred embodiments the retainers are formed through a cutting method. The retainer is formed by cutting into the body of the suture filament, providing a raised portion that includes a base where the undersurface of the retainer meets the filament body and an opposing tip that is raised from the filament body. The tip may be pointed or it may be blunted. The retainer has a first side portion and a second side portion with each side portion converging from the base to the tip, and having the body of the retainer disposed therebetween. The retainers have a generally asymmetric shape and configuration. As used herein, the term "asymmetric" as it refers to a retainer means that one half of the retainer body is sized, shaped or configured different than the second half of the retainer body, and where each half is formed by a "retainer dividing plane". A retainer dividing plane is an imaginary plane taken along a cross-section of the suture along its axial length, dividing the retainer in two segments, starting at the tip of the retainer and ending at the center of the retainer base. Details describing the retainer dividing plane will be provided below, but to be considered "asymmetric", a first half of the retainer (as separated by the retainer dividing plane) is different than the second half of the retainer. In some embodiments, only the retainer tip region is asymmetric, and in other embodiments, the retainer base is asymmetric, while in other embodiments the entire retainer is asymmetric. An asymmetric retainer has, for example, a first side portion and a second side portion that are different lengths, shapes, configurations, or that are axially off-set from each other along the suture axis.

As explained, the retainers in the suture body are produced with an asymmetric design, such as at the tip region or at the entire retainer, beginning at the tip and extending to the base. The retainers have a semi-axial arcuate base aspect. That is, the base of the retainer, where the undersurface of the retainer meets the cut body of the suture, has an arcuate shape, which may be concave towards the proximal end of the suture or concave towards the distal end of the suture. This may be achieved through the use of a rotary cutting element cutting into the body of the suture body. In preferred embodiments, the cut is initiated at a position located on the side of the filament body at the intended base location of the retainer , thereby producing a retainer geometry that is formed with a "furrowed" shape on the entry side of the suture body that exits the surface of the suture body on an opposing side.

Additionally, the cutting device exits the opposite side of the suture body at an exit location, the exit location defining the side of the retainer on the opposite side of the entry location. The exit location may be located in a position that is vertically offset (with respect to the axial length of the suture body, the x-y axis) relative to the initiation point as a result of the centerline orientation of the rotary cutting device being oriented in a skewed position relative to the central vertical axis (or z-axis) of the suture. The cutter and the rotation of the cutter will be explained in greater detail below.

The self-retaining suture provided by the present invention includes at least one and more desirably a plurality of asymmetric retainers disposed along its axial length, providing a number of benefits over not only conventional sutures but also over self-retaining sutures made by conventional methods, such as by commencing a retainer cut at the tip of the retainer and ending the cut at the base of the retainer. The self-retaining sutures of the present invention provide a suitable tensile strength to allow for pulling through tissue, but also a suitable holding strength when pulled in the opposite direction (e.g., toward the distal end of the suture). In addition, the self-retaining suture of the present invention provides visual, tactile or other feedback during use, such as when it is being pulled through the tissue.

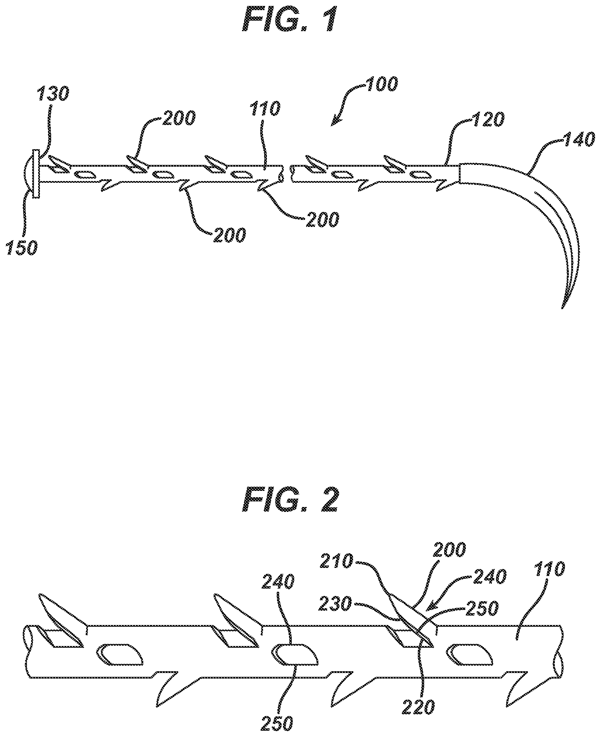

With reference to the Figures, the present invention may be better understood. Figures 1-4 show a self-retaining suture of one embodiment of the present invention. The self-retaining suture of these Figures includes a plurality of retainers spaced along at least a portion of the axial length of the suture body. It will be noted that the retainers of this embodiment are arranged in a substantially helical configuration along the body, but the retainers need not be helical and may be arranged in other configurations, including, for example, a straight line, in opposing straight lines, or in a random orientation. It is also noted that the Figures show unidirectional sutures, but the self-retaining sutures of the present invention may be bidirectional if desired.

A self-retaining suture 100 is depicted. The suture 100 has a body 110 which may be of any geometric configuration (measured by its cross-section), including circular, oval, triangular, rectangular, or any other cross-sectional shape. The suture 100 may be subjected to a pre-process whereby the suture is passed through a heating apparatus, such as a series of heated rolls or platens, and may be subjected to various tension conditions if desired. The heating and/or tension may be useful in converting the body 110 to any desired cross sectional shaped fiber in preparation for the retainer application process Although the Figures described herein show a suture 100 having a triangular cross-section, any cross-section may be used.

The suture 100 has an axial length as measured from its proximal end 120 to its distal end 130. The proximal end 120 may include a means for insertion of the suture 100 into the tissue of a body, such as a needle 140. If desired, the distal end 130 may include a termination feature or anchor 150 for stopping the pull of the suture through tissue once the suture 100 has been fully inserted into tissue. The termination feature 150 may be produced to provide a large (relative to the cross-sectional diameter of the suture body 110) bearing surface oriented perpendicular to the central axis 160 (the z-axis) of the suture 100 as illustrated, or may be produced with a termination feature oriented with the large bearing surface parallel to the axis of the fiber, or with a termination that provides an engagement feature that the suture may be passed back through Any desired termination feature 150 may be used, including, for example, a tab, button, knot, ball, bar, loop, hook, and the like.

The body 110 of the self-retaining suture 100 includes at least one and more desirably a plurality of retainers 200 arranged along a portion of its axial length. Figure 1, for example, shows retainers 200 extending from the proximal end 120 to the distal end 130, but the retainers 200 need not be included along the entire length of the suture 100. One or more regions that are retainer-free may be provided, and retainer-free sections may be disposed at the proximal end 120, distal end 130, or anywhere in between the ends 120/130. There may be more than one section of the suture 100 that is free of retainers 200, if desired. Differing sections, such as those including retainers 200 or those free of retainers 200, may be marked for recognition by either a human (e.g., visual or tactile markings) or by other computer readable or mechanical means (e.g., radiopaque markings or computer readable code).

Retainers 200 may be provided in any desired location on the suture body 110. For a suture having a triangular or other angled cross-section (such as square, rectangular, and other shapes having distinct angles), the retainers 200 may be cut into the body 110 at an apex or angle. For example, for a triangular suture, retainers 200 may be cut across one of the three apices of the triangle. For rectangular sutures, the retainers 200 may be cut across one of the four corners of the rectangle. For a cylindrical suture, the retainers 200 may be cut at any location relative to the radius of the circular cross-section. Retainers 200 may be off-set from each other along the central axis 160. Thus, for a triangular suture, a first retainer 200 may be disposed on a first apex, the second retainer 200 (the "second" retainer 200 being adjacent the first retainer 200 with respect to the central axis 160) may be disposed on the second apex, and the third retainer 200 disposed on the third apex of the triangular cross-section. This provides for a helical-type arrangement of retainers 200. The same arrangement would be applicable for other geometric cross-sections if a helical configuration is desired. Retainers may be offset by any degree desired, and may, for example, be off-set by 120°, 90°, 60°, 45° or any desired degree.

It is contemplated to form a suture having a circular or oval cross section by first forming a suture having a triangular cross section and forming retainers 200 at each apex, as described above. Starting with a triangular cross-section allows the formation of retainers 200 as described above. The resulting triangular self-retaining suture may then be formed into a different cross-sectional configuration by roll forming the suture into the desired geometry. For example, the resulting triangular suture with retainers 200 cut therein may be passed through a roll forming process to convert it to one having a round cross sectional configuration, or an oval cross-sectional configuration. In this way, the retainers are formed by using a triangular cross sectional fiber to give desired retainers, but then the suture is converted to a more typical cross-section, such as circular or oval in cross-section. This round/oval cross-sectional suture can be processed (such as needling / packaging processes) in a typical fashion as for a circular self-retaining suture, while providing improved barb geometries and tissue interactions that differ from a cut circular suture.

Each retainer 200 includes a distal tip 210, which represents the termination point of the retainer 200 and which is intended to engage the tissue during use. The retainer tip 210 faces axially away from the proximal end 120 of the suture 100 in a unidirectional device or faces away from one insertion end in a multi-directional suture device. The retainer 200 also includes a base 220, which is the intersection of the undersurface 230 of the retainer 200 and the cut surface 270 of the suture body 110. The retainer 200 also includes a first side portion 240 and a second side portion 250. Each of the first side portion 240 and second side portion 250 start at opposing ends of the base 220 and converge at the tip 210, with the body of the retainer 200 located between each side 240/250. The tip 210 may be pointed or it may be blunted. Since the retainer 200 is formed by cutting into the suture body 110, the shape of the retainer 200 will be similar to the shape of the cut-out of the suture body 110, with a vertical and lateral deflection, allowing the retainer 200 to lie flat within the cut portion of the suture body 110 when passing through tissue. Thus, the undersurface 230 of the retainer 200 will be substantially matched in size, shape and orientation with the cut surface 270 of the suture body 110. The retainer 200 may be capable of flexing or moving with respect to the suture body 110 upon the application of force.

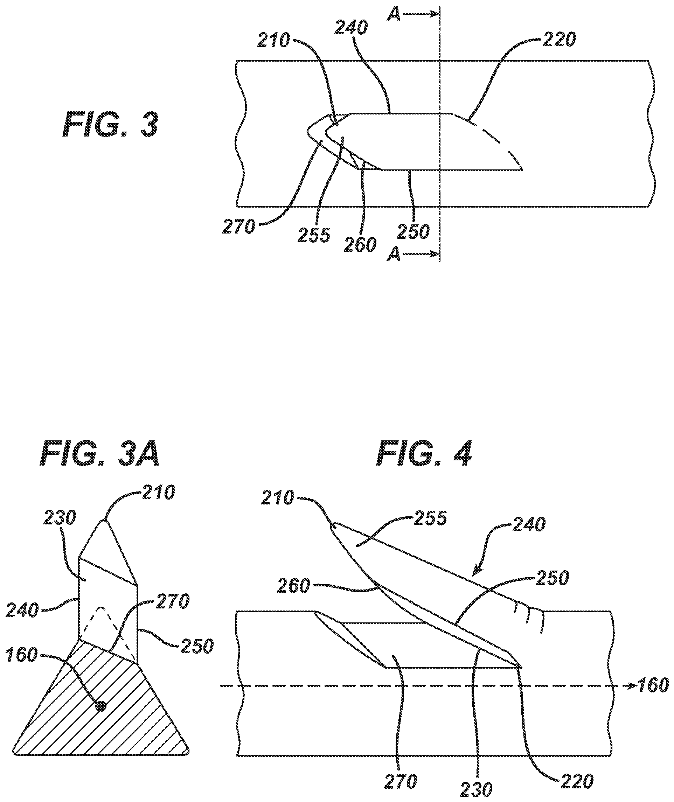

Figures 3, 3A and 4 closely show the shape and configuration of one type of retainer 200 of the present invention. The retainer 200 embodied in these Figures has a tip 210, base 220, undersurface 230, first side portion 240 and second side portion 250. As can also be seen in these Figures, the suture body 110 has been cut to leave a cut surface 270. The shape of the cut surface 270 is similar in size, shape and orientation to that of the undersurface 230. In Figures 3, 3A and 4, the retainer 200 is cut into one apex of a triangular suture 100, but as described above, any cross-sectional configuration of suture 100 is contemplated.

In some embodiments, the undersurface 230 of the retainer 200 may have more than one angle with respect to the central axis of the suture body 110. As seen in Figure 4, the retainer 200 may have an undersurface 230 that changes angles at an angle line 260. The angle of the cut surface 270 of the retainer 200 is determined with respect to the central axis 160 of the suture body 110 and may be from about 12 to 14 degrees. In some embodiments, the passage of the thickness of the cutting blade through the body may result in the angle of deflection of the undersurface 230 at a location closer to the base 220 being less than the angle of deflection of the undersurface 230 at a location closer to the tip 210. Thus, as the retainer 200 extends from the base 220 to the tip 210, the angle of deflection may become greater. This provides a retainer 200 with an angled tip region 255. As can be seen in Figure 3A, the angle of the cut (which can be seen with reference to cut surface 270) may be any desired angle across from the first side portion 240 to second side portion 250, as determined with respect to the central axis of the suture body 110. This angle cut may be useful in providing asymmetric suture retainers 200.

As can be seen in Figure 3, the retainer 200 is asymmetric (as determined by dividing the retainer by a retainer dividing plane along the axis of the suture 160 from the tip 210 to the center of the base 220, and comparing each side). The first side portion 240 of the retainer 200 may be axially off-set from the second side portion 250 with respect to the central axis 160 of the suture body 110. In addition or alternatively, the first side portion 240 may be shorter in length than the second side portion 250. This is caused by the cutting process, which will be described below. In one particularly useful cutting process, the tip of a cutting blade enters the suture body 110 at the base 220 on the second side portion 250, and the blade edge extends through the suture body 110, exiting at the first side portion 240 and at the tip 210. Given the degree of rotation of the cutting apparatus and the entry/exit points, the retainer 200 has a resulting asymmetric shape. Again, this asymmetric shape may be seen, for example, in the top view depicted in Figure 3, wherein the first half of the retainer (including the first side portion 240) has a different sizing and spacing than the second half of the retainer (including second side portion 250). Further, if an angled cut is used (such as seen in Figure 3A), the two sides of the retainer 200 (including first side portion 240 and second side portion 250) may have different configurations and sizes.

In an alternate embodiment, a cut may result in a furrowed configuration. The formation of a furrowed configuration may be useful if a fully cut retainer 200 is not desired, but rather a configuration that increases friction during the insertion of a suture 100 through tissue. The furrowed configuration provides drag when the suture 100 is pulled in a direction opposite that of the insertion direction. The furrowed suture is not intended to securely hold the suture 100 in place, as would a fully cut retainer 200, but rather to provide frictional drag when the suture 100 is pulled through tissue. Frictional drag may provide some degree of tension and hold, or may provide tactile or other sensible feedback to the user. In some embodiments, a flap may be formed in the suture body 110, which may be formed by using an incomplete penetration cut, therefore avoiding a fully cut retainer 200 but forming a flap of suture material that may provide drag.

Although furrows and flaps may be provided by avoiding a fully cut retainer and are useful in various embodiments, in the most desirable embodiment, a full retainer 200 (or plurality of retainers) is cut into the suture body. In some embodiments, the axial length of the suture may be from approximately 15.2 cm (6 inches) to about 137.2 cm (54 inches), but shorter or longer lengths may be used if desired. The suture body 110 may have a thickness of about 0.102 mm (0.004 inches) to about 1.016 mm (0.040 inches) as measured by the diameter of a cross-sectional plane perpendicular to the central axis 160 of the suture 100. Any diameter suture body 110 may be used, including those that are conventional in the art, such as those having sizes commonly referred to as 11-0, 10-0, 9-0, 8-0, 7-0, 6-0, 5-0, 4-0, 3-0, 2-0, 0, 1, 2, 3, 4, 5, 6, or 7.

The length of a retainer 200 may be from about 0.254 mm (0.010 inches) to about 1.524 mm (0.060 inches), and in some embodiments may be from about 0.508 mm (0.020 inches) to about 1.270 mm (0.050 inches) (as measured along its undersurface from the tip 210 to the center of the base 220). The length of a first side portion 240 may be approximately 0.762 mm (0.030 inches) and the length of a second side portion 250 may be approximately 1.016 mm (0.04 inches) (as measured from the tip 210 to the base 220). In some embodiments in which the undersurface 230 includes more than one angle, thereby giving the undersurface 230 at the tip region 255 a different angle than the rest of the retainer 200. In this embodiment, the shift in angle occurs at the angle line 260. If desired, the length of the first side portion 240 may be measured from the base 220 to the angle line 260 and the length of the second side portion 250 may be measured from the base 220 to the angle line 260. The base 220 may have an approximate arc length of 0.178 mm (0.007 inches) with a 11.049 mm (0.435 inch) radius cutter tip and triangular suture height of approximately 0.356 mm (0.014 inches) and with the arc length as measured by a straight line taken from the intersection of the base 220 and the first side portion 240 to the intersection of the base 220 and the second side portion 250. The sizes identified above are approximates and may vary as desired and to suit the intended needs of the suture to be formed. As will be explained below, the base 220 has an arcuate shape.

The first side portion 240 and second side portion 250 may be any desired lengths measured from the base 220 to tip 210, such that the second side portion 250 is longer than the first side portion 240. The relative ratios of lengths of the first side portion 240 to the second side portion 250 may be from about 1:1.01 to about 1:2.0, or from about 1: 1.1 to about 1:1.5.

In addition to the retainer 200 being asymmetric, the retainer tip region 255 may be asymmetric due to an angled cut across the suture body 110. This occurs as the first side of the retainer tip region 255 is shorter than the opposing side of the retainer tip region 255. The asymmetric retainer tip region 255 may be formed by cutting with an elevating surface due to cutting apparatus pivoting about an offset angled axis, as seen in Figure 3A. Additionally, the retainer 200 may be produced with the second side portion 250 being a slightly deflected edge or rolled edge. The rolling of an edge may occur as a result of a cutter blade entering the second side portion 250 and, as the depth of the cutting tip increases in the suture body 110, material may be displaced to provide space for the volume of the cutter blade. A second side portion250 having a rolled edge is an optional feature that may be included in the retainer 200 if desired.

Figure 3 also shows one other feature of the retainer 200, namely an arcuate base 220. As can be seen, the retainer 200 includes a base 220 that has an arcuate shape. This arcuate shape can be formed by the cutting process described herein, and particularly due to the rotation and the rotational speed of the cutting blade. As the blade cuts into the retainer 200 (again, it is understood and noted that the tip of the blade begins at the base 220 and cuts up through to the tip 210), the blade has a degree of rotation that forms an arcuate base 220. The arcuate base 220 may have any degree of arc, which may vary depending upon the radius of the cutting blade, which may have a radius of approximately 11.049 mm (0.435 inches). The arcuate base may also be produced with a spiral radius of origin. This effect is created through the production of the retainers while the fiber travels axially at a linear velocity that is less than the angular velocity of the rotational cutter tip during the cutting stage.

Thus, the retainers 200 of the present invention may have a number of different characteristics, each of which may be included or not included and each of which can be formed through the cutting method described below. Each retainer has an asymmetric shape, as determined by a plane extending from the tip 210 to the middle of the base 220. Thus, the retainer 200 has a first side portion 240 that is shorter than the second side portion 250 and may be off-set from the second side portion 250 with respect to the central axis of the suture body 110. Each retainer 200 may have an angled tip region 255, including an angle line 260. Each retainer has an arcuate base 220. The retainers may have the asymmetric shape and angled tip region 255. The retainers have an asymmetric shape and arcuate base 220. The retainers may have an angled tip region 255 and arcuate base 220. The retainers may have asymmetric shape, an angled tip region 255, and arcuate base 220.

In some embodiments, a retainer 200 may be formed by a linear cutting method, in which the cutting device includes a blade with a tip, and the tip of the cutter enters at the base position of the retainer 200, and traverses at an angled disposition relative to the x-y-z axis. Such linear cutting may take place while the suture 100 is held stationary in place during the cutting. The suture and/or the cutting device may then be axially moved to a different location for another cut to be made. In this embodiment, the resulting retainer 200 may have an asymmetric configuration, but may not include the arcuate base, described above. Of course, if the suture material is moving along its central axis during the time that a linear cutting blade enters the suture body, some degree of arc may be formed.

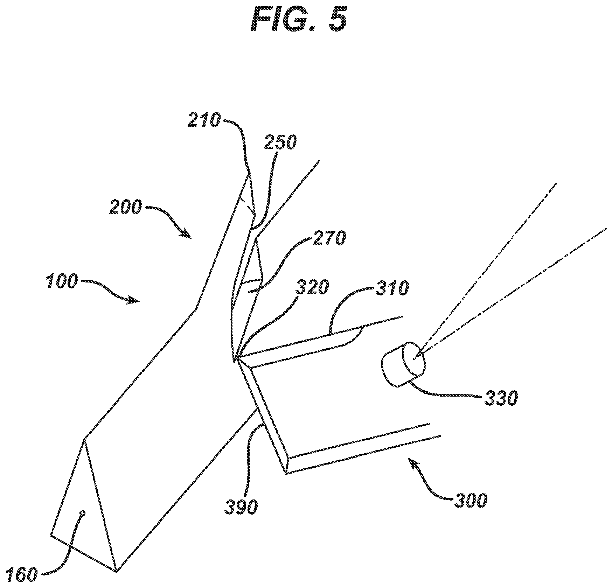

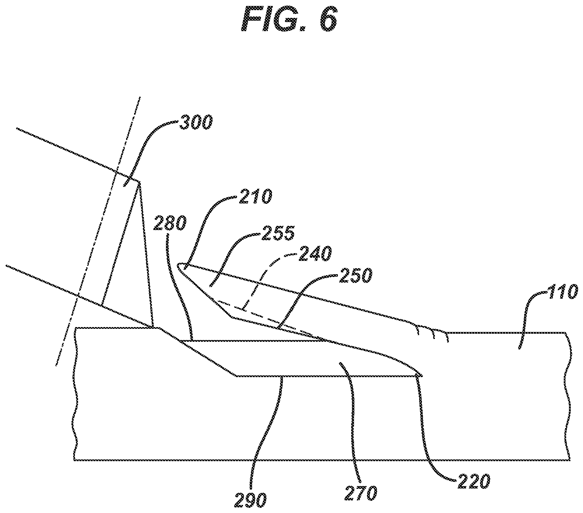

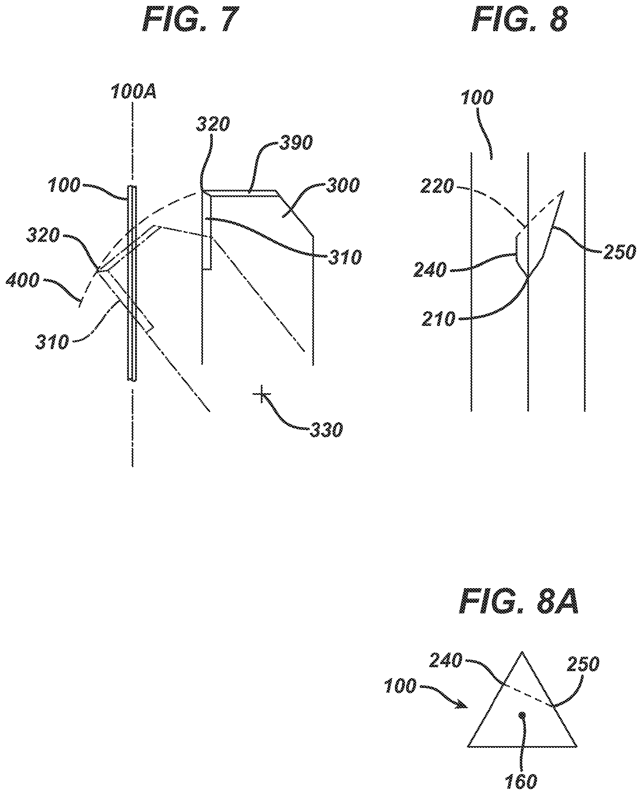

Figures 5-10 shows a cutting device 300 as it may be used to form retainers 200 in the suture body 110. Cutting device 300 includes a blade edge 310 and blade tip 320, and a pivot pin 330 about which the blade edge 310 rotates. The blade tip 320 may rotate about the pivot pin 330, and enter the suture body 110 at the base 220 of the retainer 200. As the cutting device 300 rotates about the pivot pin 330, the blade edge 310 cuts into the suture body 110, extending through the suture body 110 forming the retainer's first side portion 240, second side portion 250 and tip 210. The cutting forms the retainer 200, but also forms the cut space in the suture body 110, including the cut surface 270 (which is substantially aligned with the undersurface 230 of the retainer 200), first side of cut surface 280 (which is substantially aligned with the first side portion 240 of retainer 200) and second side of cut surface 290 (which is substantially aligned with the second side portion 250 of retainer 200). After formation, the retainer 200 may be compressed toward the center of the suture body 110 and will partially fit in the cut space in the suture body 110. This allows for insertion through tissue while providing some degree of tactile or visual feedback of tissue passage.

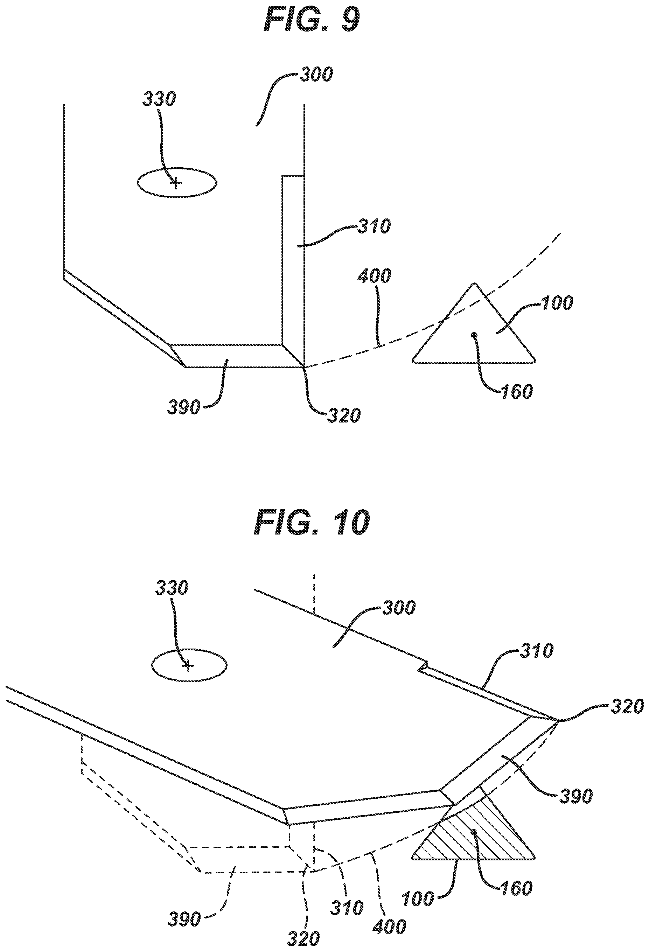

As can be seen in the Figures, the cutting device 300 may, if desired, be produced with two beveled cutting edges 310 and device 300 in Figure 5), forming the angled tip region 255 as described above (defined by angle line 260). As can best be seen in Figures 9-10, the cutting edge 310 and cutting tip 320 may rotate in a plane that extends away from the central axis 160 of the suture 100. The angled tip region 255 may be formed as a result of using multiple cutting edges (310, 390) of a cutting device 300. In the Figures, the intended starting point of the cutting device 300 is seen in phantom lines, and an exemplary path of the cutting tip 320 can be seen as dashed line 400.

As explained above, the retainers 200 in the above embodiments are cut into the suture body 110 through the use of a rotating cutting device 300 including a cutter edge 310 and cutting tip 320. The use of the rotating cutting device 300 results in the production of a retainer that includes an arcuate base 220. Additionally, the rotating cutting device 300 may be mounted on an axis that is offset from the a normal line of the suture 100 and may be mounted with an axis that is angled relative to the central axis 160 of the suture 100 to be cut. The offset position and angled central axis results in the cutter tip 320 moving in an arced path of travel toward the side of the suture body 110 to be cut instead of striking the body 110 in a tangential orientation. Additionally, due to the offset and angular positioning of the rotary axis relative to the normal axis of the suture body 110, the cutter tip 320 moves upward relative to the elevation of the suture body 110 to be cut. Further, due to the use of a rotary cutting action, the cutter tip 320 traverses in a direction that moves from the base 220 of the retainer 200 towards the tip 210 of the retainer 200.

Therefore, unlike traditional cutting methods, the method described herein actually uses a cutting device in which the blade tip/blade edge enters the suture body 110 at a location closer to the central axis 160 and exits the suture body 110 at the surface of the suture body 110 (forming the tip 210). This method is unlike traditional cutting methods which traditionally traverse from a tangential entry location forming the retainer tip first, and terminate closer to the core region of the suture body 110 to be cut, forming the retainer base after the tip is formed. Unlike traditional cutting methods, the exiting of the cutting edge at the tip location results in a slight displacement of material, or forms an edge that is slightly rolled, distally and away from the central axis of the suture body.

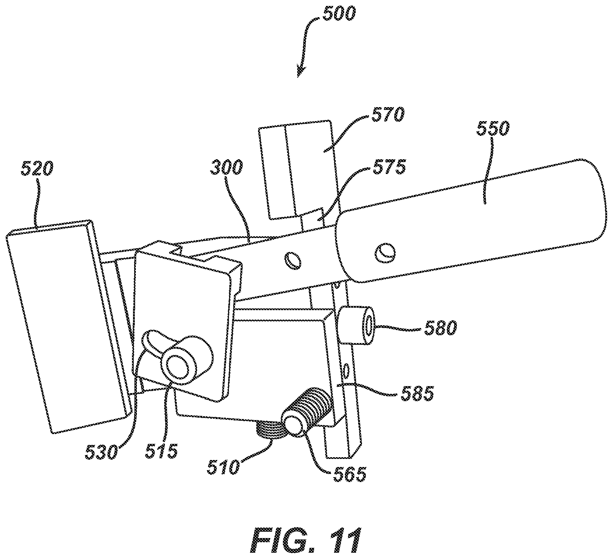

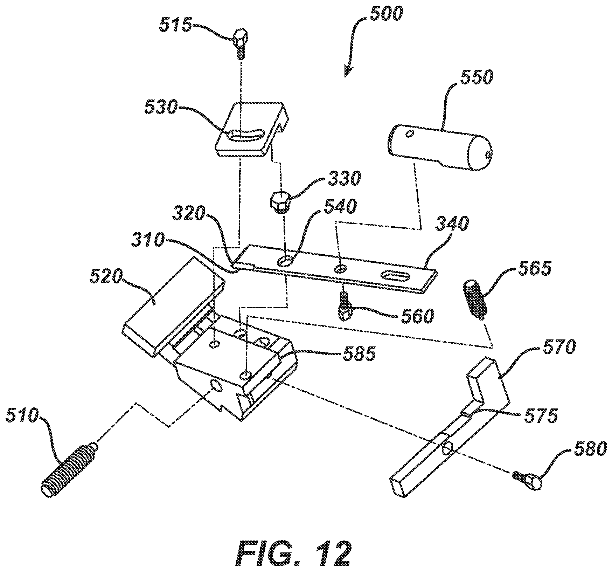

Figures 11-14 show one embodiment of a cutting device described herein, and depict a manual cutting device 500 useful for forming asymmetric retainers in the body of a suture 100. This embodiment includes a hand operated rotary cutter apparatus 500 including a shoe 520 that is designed to fit over a fiber guide rail 600, and a cutter blade pivot platform 585. The pivot platform 585 is designed to receive the cutting device 300 (having cutting edge or blade 310) via pivot hole 540 and pivot cam 330. The cutting device 300 is maintained in close proximity to the surface of the pivot platform 585 through the pivot cam 330, the pivot cam guide 530 and the pivot cam guide fastener 515. A finger handle 550 is attached to the cutting device 300 to enable the operator to rotate the device 300 to a pre-cut locked and energized position. A latch bar 570 is produced with a cutter blade engagement notch 575. The latch bar 570 may be attached to the side of the pivot platform 585 through the use of the latch bar fastener 580. A spring loaded ball plunger 565 may be inserted into the top of the pivot platform 585, passing through the platform 585 and engaging the top of the latch bar 570. In this position, the spring loaded ball plunger 565 maintains pressure against the latch bar 570 to enable the latch bar 570 to hold the cutting device 300 in an energized position. A cutter blade driver 510 may be inserted through the side of the pivot platform 585. In use, the plunger end of the cutter blade driver 510 engages the side of the cutting device 300 and drives the cutting blade 310 around the pivot cam 330. Due to the release of the spring, when the latch bar is released, the cutting device 300 is driven with a high degree of speed and force.

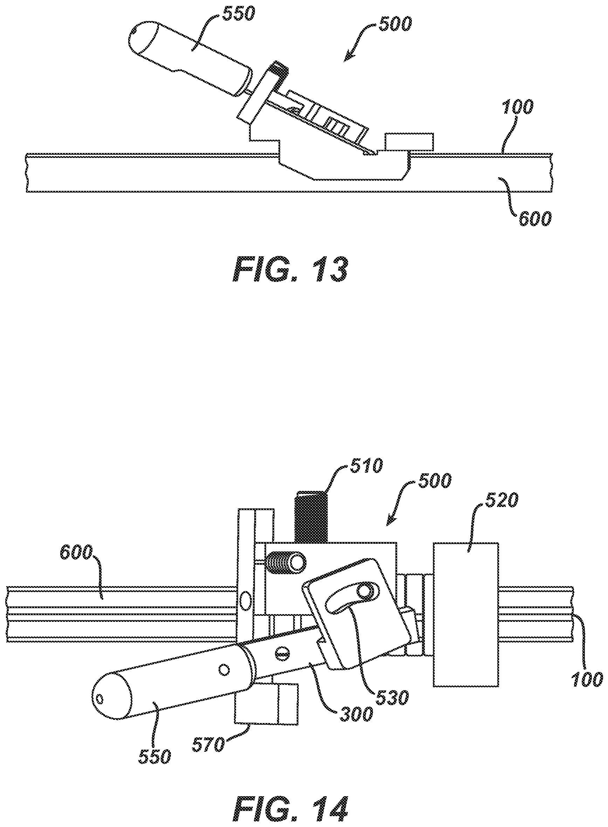

Referring to Figures 13-14, the manual cutting apparatus 500 is seen in use. A suture 100 of a desired geometry (e.g., cylindrical, triangular, rectangular, etc.) is placed upon a guide rail 600 and held in a fixed position. The manual cutting apparatus 500 is placed on top of the guide rail 600 with a relief groove on the underside of the shoe 520 located over the suture body 110. The relief groove in the shoe 520 is manufactured with a geometry and size that matches the cross sectional shape of the suture 100 to be cut. The cutting device 300 is armed through the movement of the finger handle 550 towards the axis 160 of the suture body 100 until the latch bar 570 locks. In this position, the spring pressure in the cutter blade driver 510 is at the greatest. The cutting device 300 is released by depressing wide end of the latch bar 570. Upon release, the cutting device 300 rotates rapidly about the pivot cam 330 and the cutting edge 310 strikes the side of the suture body 110. As the velocity of the cutting device 300 is significant, the cutting edge 310 is able to cut through the suture body 110 as a result of the impact of the cutting device 300 on the suture body 110.

The cutting device 300 may then be reset to the energized position again, and the manual cutting apparatus 500 may be advanced to the next location to be cut. If desired, the suture body 110 may be rotated about its central axis to a desired degree (to create helically off-set retainers 200). The guide bar 600 may then be shifted axially by a desired axial length. The degree of shifting of the guide bar 600 determines the axial location of the next retainer 200. It may be desired to shift the guide bar 600 approximately the length of one cut retainer 200 to provide spacing between adjacent retainers 200, but any desired axial spacing between retainers 200 may be used as desired. Retainers 200 may alternately be axially offset from each other by a distance of greater than one retainer, or less than one retainer length. Once in position, both axially and rotationally, the cutting device 300 may be activated, and another retainer 200 is formed in the suture body 110. If desired, the suture body 110 may be rotated again to a desired degree and the guide bar 600 may be moved axially along the length of the suture 100 to the desired axial length. Depending upon the particular fiber cross sectional shape to be cut, the degree of suture rotation can be greater or less than about 120°. For example, if a hexagonal cross sectional fiber is created; incremental rotations of about 60° may be desirable to create a greater number of rows of retainers 200 about the perimeter of the suture body 110 to emulate a spiraled disposition of the retainers 200 about the central axis of the suture 100. If a rectangular suture is used, the degree of suture rotation may be about 90°. Any degree of rotation may be used to prepare a suture 100 having the desired rotational spacing of retainers 200.

The cutting may be achieved through the use of a continuous cutting apparatus, or may include a plurality of cutting apparatuses each offset from each other by a desired radial spacing. For example, there may be three cutting apparatuses each offset from each other by 120 degrees about a suture pathway. Each cutting apparatus may be axially offset from each other or they may be disposed in the same axial plane but timed to cut into the body of a suture at different intervals. In any configuration, the timing of rotation of the cutting blades is such that the retainers will be formed having the axial and offset configuration desired. The tip of the blade 320 travels along an arc as it cuts into the body of the suture 200, thus forming an angled and/or arced cut.

In continuous processing, a suture 100 may be continuously moving in an axial direction (for example, towards the proximal or distal end of the suture) through a suture pathway in the cutting apparatus as the cutting blade or blades are in use The suture 100 may travel at a speed that is faster than the rotational speed of the blade(s) in use. Alternatively, the suture 100 may travel at a slower speed than the rotational speed of the blade(s) in use. The blade or blades in a continuous apparatus may be configured to continuously rotate about a pivot point, allowing for the blade or blades to enter the body of a suture 100 as described above, or the blades may be continuously reset to a pre-cut configuration after each cut is made. The relative speeds of blade rotation and suture movement may dictate the length or spacing of retainers, and also may dictate the level of asymmetry in the retainer, and also may dictate the radius of an arcuate base in a retainer. The suture 100 may travel at a speed of about 559 cm (220 inches) per minute in an axial direction. The blade of a rotational cutter may travel at a speed of up to about 2,300 rpm with a cutting edge tip diameter of 2.54 cm (1.0 inches). The continuous system may be used to prepare a self-retaining suture having a plurality of retainers cut into its body with speed and efficiency. Additionally, the arcuate base may be produced as a spiral form radius due to the differential velocities of the cutting tip and the suture body in motion during the cutting operation. If, for example, the suture travels at an axial rate that is faster than the speed of the cutting device 300, the resulting retainers 200 may have arcs, and vice versa. Varying the speed of the movement of the suture and/or the speed of rotational cutting of the cutting device 300 may provide retainers 200 with different sizes, shapes, and arc configurations.

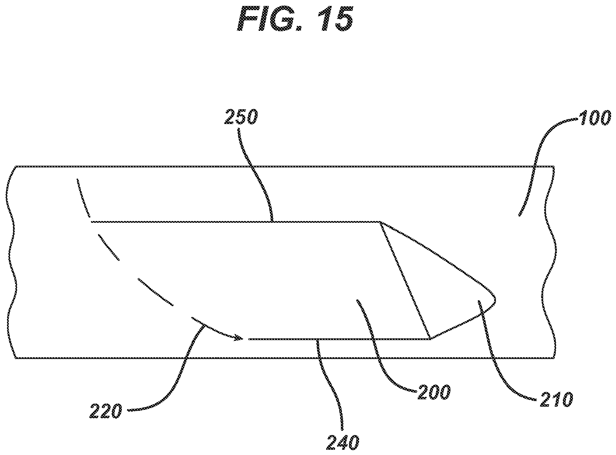

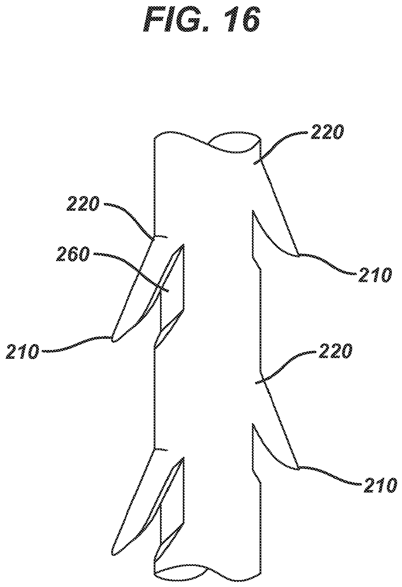

Figures 15 and 16 are photographs of a PDS triangular suture that has been cut through use of a manual cutting apparatus 500 described above. As can be seen, the resulting retainers 200 are asymmetric, include a first side portion 240 that is shorter and axially offset from the second side portion 250, include an arcuate base 220, and include an asymmetric tip region 255. Figure 16 shows that the retainers 200 also include an undersurface 230 that has more than one angle with respect to the central axis 160 of the suture body 110, having an angle line 260 where the separation of the two angles occurs. The retainers 200 of Figures 15 and 16 are offset rotationally by about 120° and are axially spaced by the length of one retainer 200. That is, the end of one retainer 200 (e.g., approximately at its base 220) is approximately aligned in the same cross-sectional plane of the suture body 110 as the beginning of the next retainer 200 (at approximately its tip 210). Longer or shorter axial spacing may be used for adjacent retainers 200, if desired.

The present invention achieves a number of benefits due to its various components. For example, an asymmetric retainer provides a lateral force to the retainer as it engages with tissue, thereby overcoming the tendency to lie within the needle track formed upon insertion through tissue. An arcuate retainer base enables the alteration of a retainer length based upon the radius of the cutter path and provides for a less distinct point of loading of the suture along an axially displaced base of the retainer, thereby distributing the load in different axial locations along the molecular chains of the polymer in the suture. A linear based retainer cut at a non-perpendicular angle relative to the central axis of the suture allows for various retainer lengths based upon the angle of axis of the cutting element during formation, and provides for a less distinct point of loading of the fiber along an axially displaced base of the retainer, much like the arcuate base. Further, modifying the angle during the cutting of the retainer may allow for multi-angled retainers, adding strength and retention capabilities. The method of cutting the retainers from the retainer base towards the distal tip of the retainer provides a number of benefits. This method not only enables the formation of the asymmetric retainer but also enables continuous cutting of the retainers on a suture when the suture is in motion. In addition, the method provides for the creation of a displacement or minor bulging of the suture surface to provide a small element of drag during tissue passage, thereby providing various cues necessary for robotic and laparoscopic surgery, such as optical and haptic cues.

With traditional sutures having substantially symmetric retainers, when the tension on the self-retaining suture is applied in the reverse direction, the symmetrically shaped retainer tip engages the tissue directly opposite the central axis of the fiber and then flexes directly outward from the central axis of the fiber as the retainers engage the tissue more fully as the tension is increased. If the tension continues to increase until the retainers release, the tissue damage that is induced prevents subsequent retainers from engaging with the tissues in the suture tract. However, with the use of the asymmetrical retainers of the present invention, the reversal of suture tension results in the retainers deflecting away from the central axis of the suture while the asymmetric shape also creates a lateral loading the retainer, thereby driving the retainer in a lateral direction from the central axis of the suture. This results in less tissue damage, and the ability of subsequent retainers to engage the tissue within the tract. In addition, by commencing the cut at the base of the retainer and ending it at the tip, various problems with conventional retainer formation methods, discussed above, can be avoided.

The self-retaining sutures of the present invention includes retainers having an asymmetric shape (as determined by dividing a retainer through the retainer dividing plane) and an arcuate base. The asymmetric shape may be throughout the retainer body or may be only at the tip region. Alternatively, the retainers may have an asymmetric shape or may have an arcuate base. The self-retaining sutures may include retainers having an undersurface that extends in more than one angle with respect to the central axis of the suture body. The self-retaining suture may include retainers having an arcuate base and have an undersurface that extends in more than one angle with respect to the central axis of the suture body. Of course, the self-retaining suture may include retainers that have an asymmetric shape, an arcuate base and an undersurface that extends in more than one angle with respect to the central axis of the suture body.

While rotary cutting methods have been disclosed, it should be noted that other methods that involve the use of linear motions whereby the cutter blade is driven from the base of the retainer towards the opposing side of the fiber being cut and exiting at a position that is closer to the barbed tip than the entrance are anticipated. Additionally, while the method described herein is illustrated through an interrupted hand driven rotary cutter blade, the cutting device 300 may be continuously rotating and timed to intersect with the suture body 110 in a continuous fashion as the suture body 110 passes the cutting device 300. The suture 100 may move at a rate that is linearly faster than the interval of the cutting device 300 timing. Additionally, it should also be noted that while the hand cutting method described herein is presented as cutting one row on one side of the suture body 110 and then subsequently rotating the suture body 110 to cut a second offset retainer, multiple cutting devices 300 mounted at offset locations about the perimeter of the suture body to be cut are contemplated.

### Examples

### Example 1 -force required to pull suture through bladder tissue

Two self-retaining sutures were tested for the force required to pull a suture through tissue. The conventional barbed suture is 2/0 V-Loc 180 (manufactured by Covidien). The inventive suture is a triangular suture, rotary cut 3/0 PDS fiber, made by the manual rotary cutter described above.

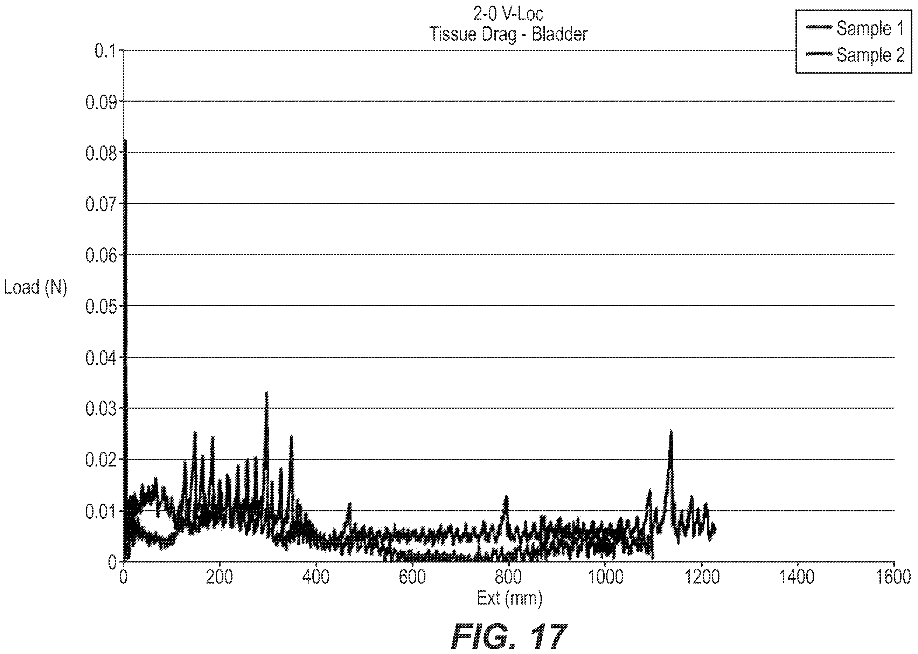

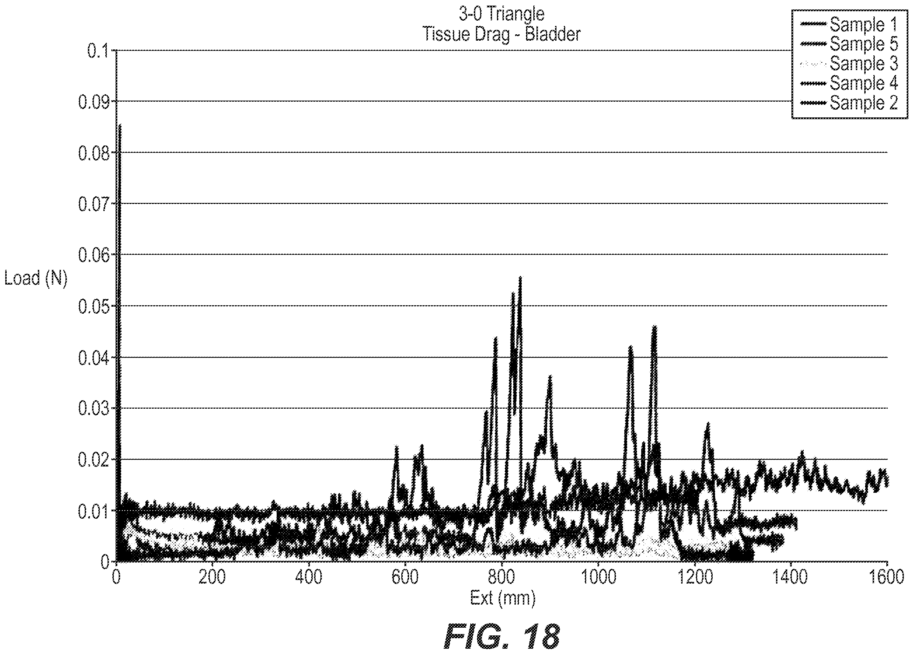

The results of the tissue interaction tests of triangular rotary cut fibers are presented in Figures 17 and 18, with Figure 17 showing the tissue drag of the conventional barbed suture and Figure 18 showing the tissue drag of the inventive self-retaining suture. Each suture was pulled through bladder tissue and the force required to pull the suture through the bladder tissue was measured. It can be seen that the conventional material produces less drag, as evidenced by the lower peak loads that are experienced as the suture is passed through the tissue. The inventive self-retaining suture produced slightly more drag, as can be seen in the Figure 18. This slight increase in average force and the ultimate peak loads experienced by the inventive suture transmits into greater tissue motion during passage. In addition, the inventive suture gives a user a slightly greater sense of tissue passage in the form of tactile feedback. Both the tactile feedback and greater tissue motion are desirable in certain environments where such cues are important, such as laparoscopic and endoscopic environments.

### Example 2 - effect of reverse tensioning

As with Example 1, two self-retaining sutures were used to measure the effect of reverse tensioning on the force required to pull the barbed suture through the tissue in the reverse direction in swine bladder. The conventional suture was 2/0 V-Loc 180 (manufactured by Covidien), and the inventive self-retaining suture was a triangular suture, rotary cut 3/0 PDS fiber, made by the manual rotary cutter described above.

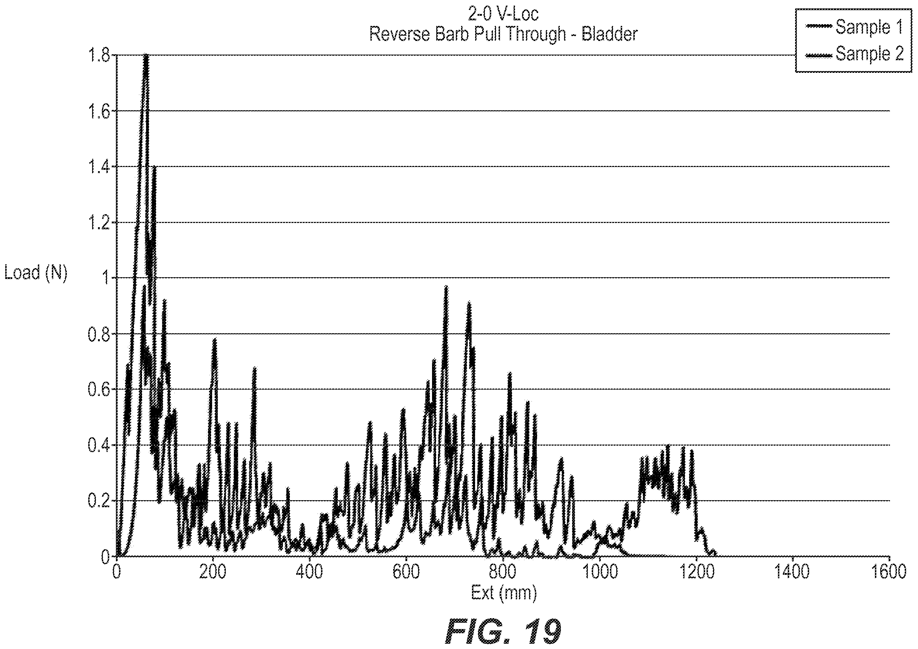

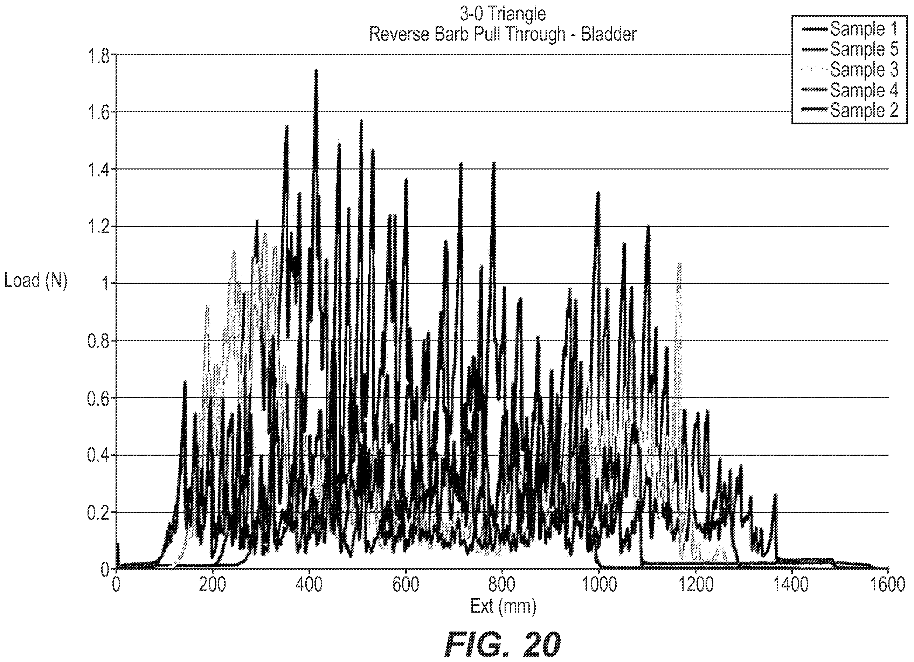

As can be seen in Figure 19, the peak loading experienced with the conventional barbed suture drops off after the initial pull through of the barbed materials. However, as can be seen in Figure 20, in the case of the inventive self-retaining suture, the repeated peak loads of the retainers as they engage the tissue during the reverse tensioning indicate greater engagement consistency of the retainers as they enter the tissue.

Based upon these results, it thus appears that the effect of the traditional cut barb as demonstrated by the conventional suture is reducing the capability of the tissues to hold the barbs in engagement as they repeatedly enter the tissue during reverse tensioning. This is evidenced by the decline in the peak loads experienced as the individual barbs enter the tissue.

In contrast, the peak load of the inventive self-retaining suture materials tend to remain elevated, thereby indicating maintenance of tissue quality to engage with the asymmetric retainers. This maintenance of engagement is favorable over traditional barbed sutures, since it appears to indicate minimal tissue damage with superior tissue loading and engagement.

## Claims

1. A self-retaining suture (100) comprising:
a. A filament having a proximal end (120) and a distal end (130), with an elongated body (110) therebetween, the elongated body having a central axis (160) and an outer perimeter; and
b. A plurality of retainers (200) formed in the elongated body of said filament, wherein each retainer has a distal tip (210), a proximal base (220) where the undersurface (230) of the retainer (200) meets the elongated body (110), a first side portion (240) connecting one end of said proximal base and said distal tip, and a second side portion (250) connecting the other end of said proximal base and said distal tip, said first side portion having a shorter length than said second side portion; and wherein said proximal base of each retainer has an arcuate shape.

2. The self-retaining suture (100) of claim 1, wherein said elongated body (110) is circular, triangular or square in cross-section.

3. The self-retaining suture of claim 1, wherein said undersurface (230) is in contact with the body of the filament when retainer (200) is pressed against filament.

4. The self-retaining suture of claim 1, wherein said undersurface (230) has more than one angle with respect to the central axis (160) of the suture body.

5. The self-retaining suture of claim 1, wherein said undersurface (230) has two angles with respect to the central axis (160).

6. The self-retaining suture of claim 1, wherein, when the retainers are formed by cutting, the cut body of the filament from which the retainer was cut has a distal end portion, and the retainer distal tip is displaced from the distal end portion of the cut body of the filament.

7. The self-retaining suture of claim 1, wherein said distal tip is pointed or blunted.

8. The self-retaining suture of claim 1, comprising at least two or at least three retainers disposed along said elongated body, optionally, wherein each retainer (200) is offset by 120 degrees with respect to the central axis along length of the filament (160), or
wherein each retainer (200) is offset by 90 degrees with respect to the central axis along length of the filament (160), or
wherein each retainer (200) is offset by 60 degrees with respect to the central axis (160) along length of the filament.

9. The self-retaining suture of claim 1, wherein said first side portion (240) has a length of about 0.762 mm as measured from the distal tip to the proximal base

10. The self-retaining suture of claim 1, wherein said second side portion (250) has a length of about 1.016 mm as measured from the distal tip to the proximal base

11. The self-retaining suture (100) of claim 1, wherein said first side portion (240) and said second side portion (250) have lengths in a ratio of from about 1:1.01 to about 1:1.5.

12. The self-retaining suture of claim 1, wherein said elongated body has a length of from 15.2 cm to 137.2 cm.

13. The self-retaining suture of claim 1, wherein said elongated body (110) includes a first region that is free of retainers and a second region that includes at least one retainer.

14. The self-retaining suture (100) of claim 1, wherein said filament includes a needle (140) secured to the proximal end (120).

15. The self-retaining suture of claim 1, wherein said filament includes an anchor at the distal end.

16. The self-retaining suture of claim 1, wherein the distal tip of each retainer faces the distal end of the suture filament.

17. The self-retaining suture of claim 1, comprising at least two retainers, where one retainer has a distal tip that faces the distal end of the suture filament and a second retainer has a distal tip that faces the proximal end of the suture filament, optionally, wherein said distal end of said filament includes a needle secured thereto.

## Patentansprüche

1. Selbstfixierendes Nahtmaterial (100), umfassend:
a. ein Filament mit einem proximalen Ende (120) und einem distalen Ende (130) mit einem dazwischenliegenden länglichen Körper (110), wobei der längliche Körper eine mittlere Achse (160) und einen Außenumfang hat, und
b. eine Vielzahl von Haltern (200), die in dem länglichen Körper des Filaments ausgebildet sind, wobei jeder Halter eine distale Spitze (210), eine proximale Basis (220), an der die Unterfläche (230) des Halters (200) mit dem länglichen Körper (110) zusammentrifft, einen ersten Seitenabschnitt (240), der ein Ende der proximalen Basis und die distale Spitze verbindet, und einen zweiten Seitenabschnitt (250) hat, der das andere Ende der proximalen Basis und die distale Spitze verbindet, wobei der erste Seitenabschnitt eine kürzere Länge als der zweite Seitenabschnitt hat und wobei die proximale Basis jedes Halters bogenförmig ist.

2. Selbstfixierendes Nahtmaterial (100) nach Anspruch 1, wobei der längliche Körper (110) einen kreisförmigen, dreieckigen oder quadratischen Querschnitt hat.

3. Selbstfixierendes Nahtmaterial nach Anspruch 1, wobei die Unterfläche (230) in Kontakt mit dem Körper des Filaments ist, wenn der Halter (200) gegen Filament gedrückt ist.

4. Selbstfixierendes Nahtmaterial nach Anspruch 1, wobei die Unterfläche (230) mehr als einen Winkel bezüglich der mittleren Achse (160) des Nahtmaterialkörpers hat.

5. Selbstfixierendes Nahtmaterial nach Anspruch 1, wobei die Unterfläche (230) zwei Winkel bezüglich der mittleren Achse (160) hat.

6. Selbstfixierendes Nahtmaterial nach Anspruch 1, wobei die Halter durch Schneiden gebildet werden, wobei der geschnittene Körper des Filaments, aus dem der Halter geschnitten worden ist, einen distalen Endabschnitt hat und die distale Halterspitze von dem distalen Endabschnitt des geschnittenen Körpers des Filaments versetzt ist.

7. Selbstfixierendes Nahtmaterial nach Anspruch 1, wobei die distale Spitze spitz oder abgestumpft ist.

8. Selbstfixierendes Nahtmaterial nach Anspruch 1, umfassend mindestens zwei oder mindestens drei Halter, die entlang des länglichen Körpers angeordnet sind, optional wobei jeder Halter (200) entlang der Länge des Filaments (160) um 120 Grad bezüglich der mittleren Achse versetzt ist oder
wobei jeder Halter (200) entlang der Länge des Filaments (160) um 90 Grad bezüglich der mittleren Achse versetzt ist oder
wobei jeder Halter (200) entlang der Länge des Filaments um 60 Grad bezüglich der mittleren Achse (160) versetzt ist.

9. Selbstfixierendes Nahtmaterial nach Anspruch 1, wobei der erste Seitenabschnitt (240), gemessen von der distalen Spitze zu der proximalen Basis, eine Länge von ungefähr 0,762 mm hat.

10. Selbstfixierendes Nahtmaterial nach Anspruch 1, wobei der zweite Seitenabschnitt (250), gemessen von der distalen Spitze zu der proximalen Basis, eine Länge von ungefähr 1,016 mm hat.

11. Selbstfixierendes Nahtmaterial nach Anspruch 1, wobei der erste Seitenabschnitt (240) und der zweite Seitenabschnitt (250) Längen in einem Verhältnis von ungefähr 1:1,01 bis ungefähr 1:1,5 haben.

12. Selbstfixierendes Nahtmaterial nach Anspruch 1, wobei der längliche Körper eine Länge von 15,2 cm bis 137,2 cm hat.

13. Selbstfixierendes Nahtmaterial nach Anspruch 1, wobei der längliche Körper (110) einen ersten Bereich aufweist, der keine Halter aufweist, sowie einen zweiten Bereich, der mindestens einen Halter hat.

14. Selbstfixierendes Nahtmaterial nach Anspruch 1, wobei das Filament eine an dem proximalen Ende (120) befestigte Nadel (140) aufweist.

15. Selbstfixierendes Nahtmaterial nach Anspruch 1, wobei das Filament einen Anker an dem distalen Ende aufweist.

16. Selbstfixierendes Nahtmaterial nach Anspruch 1, wobei die distale Spitze jedes Halters dem distalen Ende des Nahtmaterialfilaments zugewandt ist.

17. Selbstfixierendes Nahtmaterial nach Anspruch 1, umfassend mindestens zwei Halter, wobei ein Halter eine distale Spitze hat, die dem distalen Ende des Nahtmaterialfilaments zugewandt ist, und ein zweiter Halter eine distale Spitze hat, die dem proximalen Ende des Nahtmaterialfilaments zugewandt ist, optional wobei das distale Ende des Filaments eine daran befestigte Nadel aufweist.

## Revendications

1. Suture auto-rétentive (100) comprenant :
a. un filament ayant une extrémité proximale (120) et une extrémité distale (130), avec un corps allongé (110) entre les deux, le corps allongé ayant un axe central (160) et un périmètre extérieur ; et
b. une pluralité d'éléments de contention (200) formés dans le corps allongé dudit filament, chaque élément de contention ayant une extrémité distale (210), une base proximale (220) où la surface inférieure (230) de l'élément de contention (200) rencontre le corps allongé (110), une première partie latérale (240) reliant une extrémité de ladite base proximale et ladite extrémité distale, et une seconde partie latérale (250) reliant l'autre extrémité de ladite base proximale et ladite extrémité distale, ladite première partie latérale ayant une longueur plus courte que ladite seconde partie latérale ; et la base proximale de chaque élément de contention ayant une forme arquée.

2. Suture auto-rétentive (100) selon la revendication 1, ledit corps allongé (110) ayant une section transversale circulaire, triangulaire ou carrée.

3. Suture auto-rétentive selon la revendication 1, la surface inférieure (230) étant en contact avec le corps du filament lorsque l'élément de contention (200) est pressé contre le filament.

4. Suture auto-rétentive selon la revendication 1, ladite surface inférieure (230) ayant plus d'un angle par rapport à l'axe central (160) du corps de suture.

5. Suture auto-rétentive selon la revendication 1, ladite surface inférieure (230) ayant deux angles par rapport à l'axe central (160).

6. Suture auto-rétentive selon la revendication 1, lorsque les éléments de contention sont formés par découpage, le corps du filament à partir duquel l'élément de contention a été découpé ayant une extrémité distale, et l'extrémité distale de l'élément de contention étant décalée par rapport à l'extrémité distale du corps du filament découpé.

7. Suture auto-rétentive selon la revendication 1, l'extrémité distale étant pointue ou émoussée.

8. Suture auto-rétentive selon la revendication 1, comprenant au moins deux ou au moins trois éléments de contention disposés le long du corps allongé, éventuellement, chaque élément de contention (200) étant décalé de 120 degrés par rapport à l'axe central sur la longueur du filament (160), ou
chaque élément de contention (200) étant décalé de 90 degrés par rapport à l'axe central sur la longueur du filament (160), ou
chaque élément de contention (200) étant décalé de 60 degrés par rapport à l'axe central (160) sur la longueur du filament.

9. Suture auto-rétentive selon la revendication 1, la première partie latérale (240) ayant une longueur d'environ 0,762 mm, mesurée de l'extrémité distale à la base proximale.

10. Suture auto-rétentive selon la revendication 1, la seconde partie latérale (250) ayant une longueur d'environ 1,016 mm, mesurée de l'extrémité distale à la base proximale.

11. Suture auto-rétentive (100) selon la revendication 1, ladite première partie latérale (240) et ladite seconde partie latérale (250) ayant des longueurs dans un rapport d'environ 1:1,01 à environ 1:1,5.

12. Suture auto-rétentive selon la revendication 1, ledit corps allongé ayant une longueur comprise entre 15,2 cm et 137,2 cm.

13. Suture auto-rétentive selon la revendication 1, ledit corps allongé (110) comprenant une première région exempte de éléments de contention et une seconde région comprenant au moins un élément de contention.

14. Suture auto-rétentive (100) selon la revendication 1, ledit filament comprenant une aiguille (140) fixée à l'extrémité proximale (120).

15. Suture auto-rétentive selon la revendication 1, ledit filament comprenant un ancrage à l'extrémité distale.

16. Suture auto-rétentive selon la revendication 1, l'extrémité distale de chaque élément de contention faisant face à l'extrémité distale du filament de suture.

17. Suture auto-rétentive selon la revendication 1, comprenant au moins deux éléments de contention, un élément de contention ayant une pointe distale qui fait face à l'extrémité distale du filament de suture et un deuxième élément de contention ayant une pointe distale qui fait face à l'extrémité proximale du filament de suture, éventuellement, ladite extrémité distale dudit filament comprenant une aiguille fixée à celle-ci.
